# EUROPEAN PATENT SPECIFICATION

(11) **EP 2 822 504 B1**
(45) Date of publication and mention of the grant of the patent: **16.05.2018**
(21) Application number: 13757259.0
(22) Date of filing: 07.03.2013
(51) Int. Cl.: A61F 2/06, A61F 2/915

(54) **MEDICAL DEVICE FOR IMPLANTATION INTO LUMINAL STRUCTURES**
MEDIZINISCHE VORRICHTUNG ZUR IMPLANTATION IN LUMINALE STRUKTUREN
DISPOSITIF MÉDICAL POUR UNE IMPLANTATION DANS DES STRUCTURES LUMINALES

(30) Priority: 07.03.2012 US 201261607938 P; 19.07.2012 US 201261673359 P
(43) Date of publication of application: 14.01.2015
(73) Proprietor: OrbusNeich Medical, Inc., Ft Lauderdale, FL 33309 (US)
(72) Inventor: COTTONE, Robert, J., Davie, FL 33330 (US)
(74) Representative: Jilderda, Anne Ayolt
(86) International application number: PCT/US2013/029711
(87) International publication number: WO 2013/134560

(56) References cited:
- WO-A1-02/091958
- US-A1- 2002 095 208
- US-A1- 2006 036 312
- US-A1- 2006 036 312
- US-A1- 2008 051 868
- US-A1- 2008 051 873
- US-A1- 2009 171 426
- US-A1- 2010 121 430
- US-B1- 6 461 380

## Description

### Related Applications

This application is a Nonprovisional of, claims priority to U.S. Provisional Application No. 61/607,938 filed March 7, 2012 and U.S. Provisional Application No.: 61/673,359 filed July 19, 2012.

### Field of the Invention

The present invention relates to stents. In particular, the present invention relates to geometric designs of stents, which exhibit a high degree of radial strength and flexibility.

### Background of the Invention

Stents are scaffolds, which are positioned in diseased vessel segments to support the vessel walls. During angioplasty, stents are used to repair and reconstruct blood vessels. Placement of a stent in the affected arterial segment prevents elastic recoil and closing of the artery. Stents also prevent local dissection of the artery along the medial layer. Physiologically, stents may be placed inside the lumen of any space, such as an artery, vein, bile duct, urinary tract, alimentary tract, tracheobronchial tree, cerebral aqueduct or genitourinary system. Stents may also be placed inside the lumen of non-human animals, such as primates, horses, cows, pigs and sheep US 2008/0051873 shows a prior art stent.

In general, there are two types of stents: self-expanding and balloon-expandable. Self-expanding stents automatically expand once they are released and assume a deployed, expanded state. A self-expanding stent is placed in the vessel by inserting the stent in a compressed state into the affected region, e.g., an area of stenosis. Compression or crimping of the stent can be achieved using crimping equipment (see, http://www.machinesolutions.org/stent_crimping.htm, April, 2009) The stent may also be compressed using a tube that has a smaller outside diameter than the inner diameter of the affected vessel region. Once the compressive force is removed or the temperature raised, the stent expands to fill the lumen of the vessel. When the stent is released from confinement in the tube, the stent expands to resume its original shape, in the process becoming securely fixed inside the vessel against the wall.

A balloon-expandable stent is expanded using an inflatable balloon catheter. Balloon-expandable stents may be implanted by mounting the stent in an unexpanded or crimped state on a balloon segment of a catheter. The catheter, after having the crimped stent placed thereon, is inserted through a puncture in a vessel wall and moved through the vessel until it is positioned in the portion of the vessel that is in need of repair. The stent is then expanded by inflating the balloon catheter against the inside wall of the vessel. Specifically, the stent is plastically deformed by inflating the balloon so that the diameter of the stent is increased and the stent expanded.

There are functional limitations common to many stents. These include, for example, comparative rigidity of the stent in a crimped as well as deployed state, and limited flexibility making delivery and placement in narrow vessels difficult. The present invention provides a geometric design for a stent that offers both a high degree of flexibility and significant radial strength. The design of this stent also allows it to be inserted into small diameter vessels having tortuous vascular anatomy.

### Summary of the Invention

Insofar as the terms "invention" and/or "embodiment" are used in the following, and/or features are presented as being optional, this should be interpreted in such a way that the only protection sought is that of the invention as claimed. A stent comprising at least one bioabsorbable polymer and a plurality of circumferential elements is herein disclosed. Circumferential elements that are adjacent to one another may form an adjacent pair that is connected by one or more first connection elements and one or more second connection elements. In some embodiments, the circumferential elements may be formed from undulations having a sinusoidal pattern. The first and second connection elements and portions of the circumferential elements may form a substantially helical pattern across the longitudinal axis of the stent. In one embodiment, the first connection element may include one or more radiopaque markers. In various embodiments, there may be, for example, three or six first connection elements between each pair of circumferential elements. The second connection element may be, for example, linear or curvilinear.

In some cases, the circumferential elements in each pair may be in phase with each other. In this case, the first connection element may connect a valley of one undulation in one circumferential element in a pair of circumferential elements with a peak of one undulation of a second circumferential element in the pair of circumferential elements.

In one embodiment, the second connection element may connect two adjacent pairs of circumferential elements from a valley of one undulation in one circumferential element in a first pair to a peak of one undulation in a second circumferential element in a second adjacent pair.

In some cases, the stent may be crimped, while in others, the stent may be expanded. At times, when the stent is crimped and the second connection element is curvilinear, the second connection element may become substantially linear after expansion of the stent. When the stent is expanded, the distance between adjacent pairs of circumferential elements may increase and the overall length of the stent may remain substantially constant.

Additionally or alternatively, stent consistent with the present invention may comprise a plurality of circumferential components and each circumferential component may comprise a plurality of polygons. Adjacent circumferential components of the stent may be are connected by one or more second connection elements such that a substantially helical pattern is formed along the longitudinal axis of the stent. At times, adjacent circumferential components may be connected by three or more second connection elements.

In some cases, the second connection may be curvilinear and may become substantially linear after expansion of the stent. In this case, when a distance between adjacent pairs of circumferential elements increases and the overall length of the stent may remain substantially constant. Likewise, when a distance between adjacent pairs of circumferential elements decreases, the overall length of the stent may remain substantially constant.

### Brief Description of the Drawings

The present application is illustrated by way of example, and not limitation, in the figures of the accompanying drawings, in which:
Figure 1A depicts a planar view of an exemplary stent, in accordance with embodiments of the present invention;
Figure 1B depicts a planar view of an enlargement of a selected area of the stent depicted in Figure 1A, in accordance with embodiments of the present invention;
Figure 2 depicts a planar view of an exemplary stent, in accordance with embodiments of the present invention;
Figure 3 depicts a three-dimensional illustration of an exemplary stent, in accordance with embodiments of the present invention;
Figures 4 and 5 depict a planar view of an exemplary stent highlighting a set of connection elements, in accordance with embodiments of the present invention;
Figures 6 and 7 depict a planar view of an exemplary stent with curvilinear connection elements, in accordance with embodiments of the present invention;
Figures 8A-8E depict a planar view of enlargement of a selected area of stent depicted in Figure 7, in accordance with embodiments of the present invention;
Figure 9 depicts a planar view of an exemplary stent with curvilinear connection elements, in accordance with embodiments of the present invention; and
Figures 10A-10C depict a three-dimensional view of a stent, in accordance with embodiments of the present invention.

Throughout the drawings, the same reference numerals and characters, unless otherwise stated, are used to denote like features, elements, components, or portions of the illustrated embodiments. Moreover, while the subject invention will now be described in detail with reference to the drawings, the description is done in connection with the illustrative embodiments. It is intended that changes and modifications can be made to the described embodiments without departing from the true scope of the subject invention as defined by the appended claims.

### Detailed Description of the Invention

The present invention relates to expandable stents with a geometric design that exhibits a high degree of flexibility and significant radial strength. The stents of the present invention comprise a generally cylindrically shaped main body having a plurality of expandable first and second circumferential elements. The stent may be formed from bioabsorbable polymer and comprise a plurality of circumferential elements with adjacent circumferential elements forming a pair of circumferential elements. The circumferential elements in a pair are connected by at least one first connection element and adjacent pairs are connected by at least one second connection element. Portions of both the first and second connection elements form a substantially helical pattern across the long or longitudinal axis of the stent.

When the stent is expanded, the circumferential elements form ring- or hoop-like structures. Therefore, when expanded, the stent comprises a stack of double rings with each double ring formed by a pair of expanded circumferential connection elements. The stent may further comprise an end zone that caps one or both ends of the stent.

The circumferential elements have cylindrical axes that are substantially collinear with the cylindrical axis of the main body. The circumferential elements may be substantially wavelike in form (e.g., sinusoidal) providing a series of alternating valleys and peaks. Alternatively, the circumferential elements may also take other forms, such as zigzag patterns. When a radial expanding force is applied to the stent, the circumferential elements expand radially and elongate circumferentially. Conversely, when an external compressive force is exerted on the stent, the circumferential elements contract radially and shorten circumferentially.

The circumference of the circumferential elements can be constant across the body of the stent or can vary.

A circumferential element may comprise a plurality of meandering elements, undulations or polygons. An undulation may take the shape of a stylized S, Z, L (1), M, N, W, etc. An undulation may also take any other suitable configurations. The undulations may be joined together to form a pattern. When the stent is crimped, the pattern may be repeating or non-repeating. The undulations within a circumferential element may be identical or different. For example, a circumferential element may comprise a plurality of first undulations and a plurality of second undulations. A circumferential element may also comprise a plurality of first undulations, a plurality of second undulations and a plurality of third undulations. Multiple geometric types of undulations within a circumferential element are encompassed by the invention. The number of types of undulations in a circumferential element may range from 1 to 20, from 1 to 15, from 2 to 10, or from 2 to 6.

The undulations may be joined together to form an alternating pattern or other repeating patterns. Non-limiting examples of the repeating patterns include, a sinusoidal wave-form, a square wave form, a rectangular wave form, a triangle wave form, a spiked wave form, a trapezoidal wave form and a saw-tooth wave form. The undulations in a circumferential element may also be joined together to produce a non-repeating pattern. Patterns that can be used in the present invention include any suitable pattern that enables the circumferential element to expand when a radial expanding force is exerted on the stent or collapse when an external compressive force is applied.

An undulation may be a meandering element that has at least one amplitude. The amplitude of an undulation is defined by an axial distance between a valley (or one of the valleys) and a peak (or one of the peaks) of the undulation. When a radial expanding force is applied to the stent, the undulation contracts in amplitude. Conversely, when an external compressive force is exerted on the stent, the undulation increases in amplitude. When a circumferential element contains more than one undulation, the amplitude of the undulations may be identical or different. In certain embodiments, in a circumferential element, each peak may be axially spaced a similar distance from each valley such that the undulations in the circumferential element have identical amplitude. Alternatively, the amplitude of the undulations of a circumferential element may vary.

An undulation may comprise one or more segments. The segments may be linear or curvilinear. When a segment is curvilinear, the degree of curvature may vary. A segment may be concave or convex. A segment may contain solely linear portions joined together, or solely curved portions joined together. Alternatively, a segment may contain both linear portions and curved portions that are joined together. The segment may comprise at least one bend placed at selected points along its length. For example, a segment may take the shape of a stylized n, C, U, V, etc. A segment may also be in the shape of a loop where the loop may be circular or semicircular. The segment can essentially assume any suitable configuration. The length, width and thickness of the segments of the undulations may be equal or unequal. The two undulations of each polygon across each circumferential component may be identical or may vary.

A circumferential element may contain a plurality of undulations forming a repeating or non-repeating pattern. For example, when crimped, a circumferential element may be in a sinusoidal pattern. As described above, a circumferential element may take any suitable configuration. In one embodiment, the circumferential elements may comprise a ring- or hoop-like structure when expanded where the ring or hoop is substantially in the same plane; a plane is a theoretical two-dimensional unit that is cutting substantially orthogonal to the cylindrical axis of the stent.

The filament width of the circumferential elements may range from about 0.05 mm to about 2.5 mm, from about 0.05 mm to about 1.3 mm, from about 1 mm to about 2 mm, from about 1.5 mm to about 2.5 mm, from about 0.05 mm to about 1.5 mm, from about 0.05 mm to about 1 mm, from about 0.05 mm to about 0.5 mm, from about 0.05 mm to about 0.3 mm, from about 0.08 mm to about 0.25 mm, from about 0.1 mm to about 0.25 mm, from about 0.12 mm to about 0.2 mm, about 0.15 mm about 0.18 mm, about 0.20 mm, or about 0.13 mm.

Pairs of circumferential elements may be connected by at least one first connection element, but the number of first connection elements can range from 2, 3, 4, 5, 6, 7, 8, 9 to 10 connection elements with higher numbers of connection elements also being encompassed by the present invention.

Both the first and second connection elements can assume a variety of different configurations (as used herein the terms "strut" and "connection element" are used interchangeably). The connection elements can assume a variety of angles relative to the cylindrical axis of the stent, including, 0-20°, 20-40° and 40-60° (the angle of these connection elements may be positive or negative relative to the cylindrical axis of the stent). The connection elements can be straight or curvilinear. The curvilinear connection element may be concave and convex with curvature present at selected portions of the connection element where the degree of curvature varies.

A connection element can simply be an adjoining point/region of adjacent circumferential elements. In this case, adjacent circumferential elements are directly connected.

A connection element may connect the peak of a circumferential element to the valley of an adjacent circumferential element. Alternatively, a connection element may connect the peak (or valley) of a circumferential element to the peak (or valley) of an adjacent circumferential element. However, any region of adjacent circumferential elements can be connected by a connection element.

The form, number and location of the connection elements may be adapted to result in desired stent properties. The connection elements may be any appropriate shape and may be meandering whereby the connection element varys in length through the center line of the connection element. For example, the connection elements may be linear, curved, V-shaped, S-shaped, Z-shaped, I-shaped, L-shaped, M-shaped, bent L-shaped, zig-zag-shaped, etc. A connection element may also be in the shape of a repeating or a non-repeating pattern.

The number of connection elements between two circumferential elements forming a pair or between adjacent pairs of circumferential elements may be modified to suit the flexibility of the stent. Generally, the fewer connection elements the more flexible the stent may be. When there is more than one connection element between circumferential elements forming a pair or between adjacent pairs of circumferential elements, the connection elements may be positioned symmetrically or asymmetrically at radial positions along the circumference of the stent. If the connection elements are positioned symmetrically, the radial distance between each pair of connection elements is equal. The radial positions listed for the connection elements here are only provided for illustration purposes and the connection elements may be positioned by one of ordinary skill in the art without undue experimentation at any point along the circumference of the stent. For example, the positioning of the connection elements may be determined by dividing 360° by n where n is the number of connection elements. Where n = 3, the connection elements may be positioned symmetrically at approximately 120° intervals around the circumference of the stent. When there are two equally spaced connection elements between adjacent circumferential elements, they are situated approximately 180° with respect to one another. In other words, the two connection elements are oppositely oriented with respect to one another.

The amplitude of the circumferential elements may range from about 0.2 mm to about 3 mm, from about 0.5 mm to about 2.5 mm, from about 0.5 mm to about 2 mm, from about 0.2 mm to about 2 mm, from about 0.3 mm to about 1.5 mm, from about 0.3 mm to about 1 mm, from about 0.5 mm to about 1 mm, from about 1 mm to about 2 mm, from about 1 mm to about 1.5 mm, 0.81 mm, 0.83 mm or 1.47 mm.

The stent may contain a plurality of polygons. The polygon has n-sides where n is any positive integers. For example, the polygons may have a number of sides ranging from 3 to 30 (higher order polygons are also encompassed by the designs of the present invention), e.g., 3, 4, 5,6,7,8,9,10,11,12,13,14,15,16,17,18,19,20,21,22,23,24,25,26,27,28,29 and 30 sided polygons, up to an n-sided polygon. The sides of the polygons may be equal or unequal. In some embodiments, opposite sides in a polygon are substantially parallel to each other when the stent is crimped. Opposite sides in a polygon may also take other configurations in relation to each other. Adjacent polygons are connected by at least one connection element.

The polygon may be formed from a plurality of undulations that are connected by a plurality of segments. For example, the polygon may be a hexagon formed from two undulations connected by two segments. A hexagon may comprise a first undulation and a second undulation, which are connected by a first segment and a second segment. The first and second undulations in each hexagon may have different or identical width, length and thickness. The polygon may also be formed from a plurality of undulations without connecting segments. For example, the polygons may be tetragons consisting of two undulations. In higher-order polygons, e.g., n=8-30, the undulations may be connected by a plurality of segments.

A wide variety of different configurations for the polygons as well as the various segments representing the sides of the polygon are encompassed by the present invention. For example, the segments representing the sides of the polygon may be linear or curvilinear. In one polygon, the length of the segments comprising one undulation may be equal to or greater than the length of the segments of the opposing undulation.

The polygon may be convex (i.e., all its interior angles are less than 180°) or non-convex (i.e., it contains at least one interior angle greater than 180°).

The polygons can form a continuous, interconnected structure across the circumferential component (i.e., a pair of circumferential elements forming a pair), where polygons within one circumferential element share at least one side (or at least part of one side).

A circumferential component may contain different or substantially identical polygons. The polygons of different circumferential components may be different or substantially identical. The surface area of adjacent polygons may be equal or unequal. The surface area of the polygons, i.e., the area encompassed by the sides, can be calculated mathematically from the length of the sides of the
polygon.http://mathworld.wolfram.com/PolygonArea.html,April,2009. Various embodiments of the stent encompassed by the present invention are illustrated in the following figures.

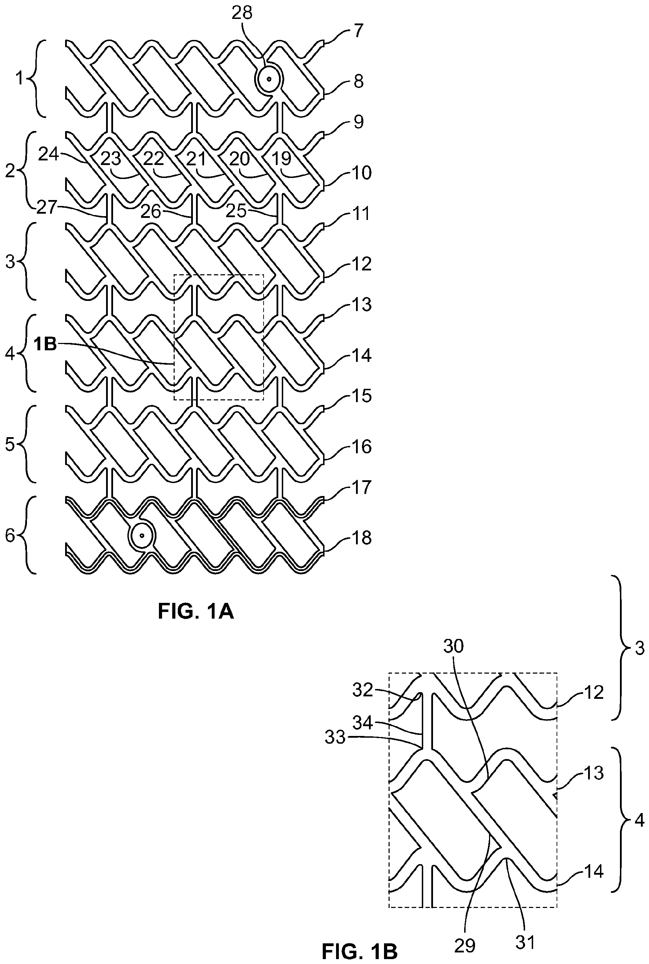

Figure 1A shows one embodiment of the stent. The pairs of circumferential elements are shown as 1-6. Each of the pairs, 1-6, is composed of two circumferential elements as follows: 1(7,8), 2(9,10), 3(11,12), 4(13,14), 5(15,16) and 6(17,18). (The numbers in the parentheses represent each individual circumferential element). Each pair, 1-6, can be connected by connection elements, first connection elements, 19-24. The number of connection elements shown in this embodiment is six, but can vary. Pairs of adjacent circumferential elements, 1-6, are connected by connection elements, second connection elements, 25-27. The stent may contain a marker dot positioned at one end of the stent, 28. Figure 1B shows an enlargement of a selected area of the stent in Figure 1A. The circumferential elements, 13, 14, in pair 4 are connected by connection element 29, from valley 30 to peak 31 in the undulations forming the circumferential elements. In the embodiment shown in this figure, the circumferential elements in each pair, 1-6, are in-phase with each other. In other words, the two patterns are superimposable and the undulations forming the circumferential elements rise and fall in the same position along the radial axis. The circumferential elements across pairs are also in-phase in this embodiment. However, in other embodiments, the circumferential patterns can be out of phase with each other, ranging from greater than 0° to 180°, e.g., 30°, 45°, 60°, 90°, 120°, or 180° out of phase. Adjacent pairs of circumferential elements, 3,4, are connected by at least one connection element, second connection element, 34, from peak to peak 32, 33.

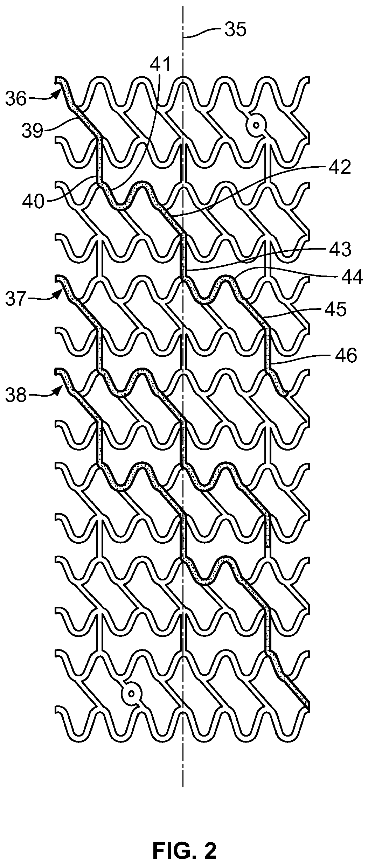

Portions of the first and second connection elements together with the circumferential elements form a substantially helical pattern along the longitudinal axis of the stent. This is graphically illustrated in Figure 2. The longitudinal axis of the stent is shown as 35. The substantially helical patterns are shown as 36-38. The helical pattern 36 is formed as follows: first connection element 39, second connection element 40, portion of circumferential element 41, first connection element 42, second connection element 43, portion of circumferential element 44, first connection element 45 and second connection element 46. The helical patterns 36-38 and are substantially parallel to each other.

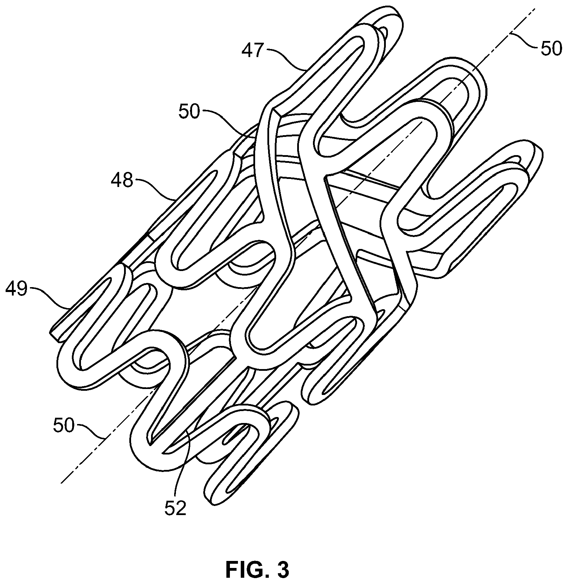

A 3D view of the stent in Figures 1 and 2 is shown in Figure 3. The circumferential elements are noted as 47, 48, and 49. A pair of circumferential elements is 47,48 and the pair is connected by a plurality of first connection elements 50. The longitudinal axis of the stent is labeled 50. Adjacent pairs of circumferential elements 48,49 are connected by a second connection element 52.

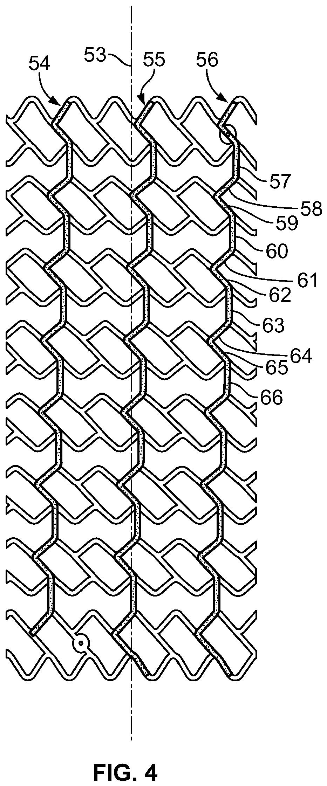

The connection elements provide structural support for the stent as illustrated in Figure 4, which shows a partially expanded flat-cut version of the stent. The longitudinal axis of the stent is labeled 53. Portions of the circumferential elements and first and second connection elements form the circumferential elements form a linear support structure 54, 55, 56, along the longitudinal axis 53 of the stent. The linear support structure is formed from first connection elements 57,60,63,66, second connection elements 59, 62, 65 as well as portions of the circumferential elements 58, 61, 64.

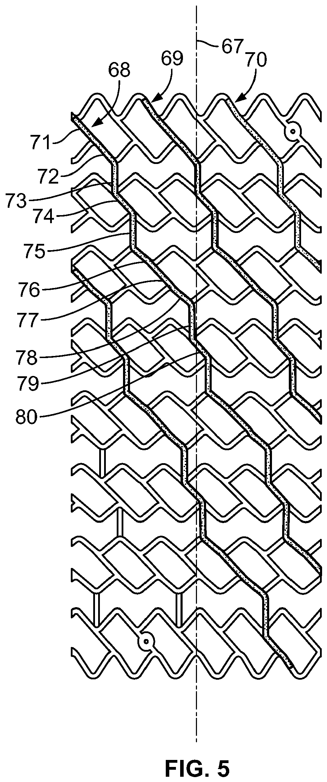

Another view of the substantially helical structures formed from first and second connection elements together with portions of the circumferential elements is shown in Figure 5. The longitudinal axis of the stent is shown as 67. The substantially parallel helical structures are shown as 68, 69 and 70. The helical structure is formed as follows, first connection element 71, portion of circumferential element 72, second connection element 73, first connection element 74, second connection element 75, portion of circumferential element 76, first connection element 77, portion of circumferential element 78, second connection element 79 and first connection element 80. Note, the sequence of connection elements interspersed with portions of circumferential elements is only provided for illustration and other sequences are encompassed by the invention.

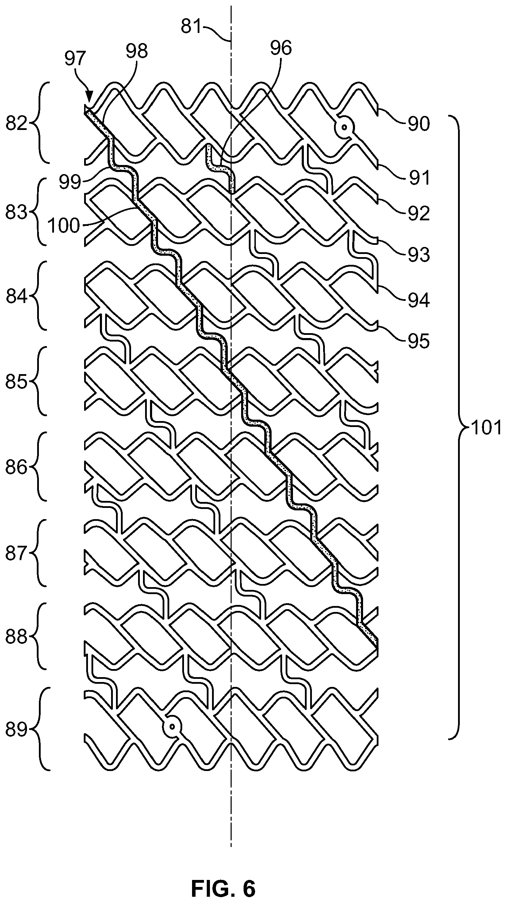

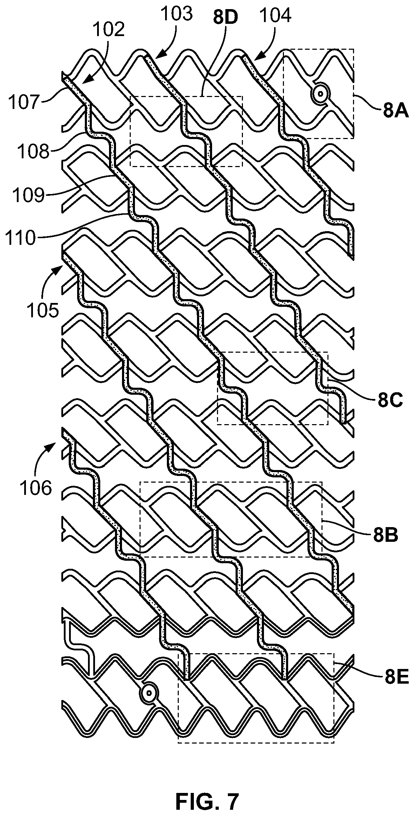

Figure 6 shows an embodiment where the connection elements between adjacent pairs of circumferential elements, the second connection elements, exhibit a different shape. In this embodiment, the second connection element, the connection element between adjacent pairs of circumferential elements, is shown as curvilinear. Specifically, the longitudinal axis of the stent is shown as 81. The pairs of circumferential elements, 82-89, are connected by curvilinear connection elements, second connection elements connecting adjacent pairs, 96. The substantially helical structure 98 is composed of a sequence of second connection elements 99, first connection elements 100, second connections elements 99 and so on throughout the body of the stent 101. The helical structure formed in this embodiment by the first and second connection elements is shown in Figure 7 102-107. The helical structures 102-107 are formed from alternating first 107, 109 and second 108, 110 connection elements across the helical structure. Of note, in this embodiment, the first connection elements connect every adjacent peak in a pair of circumferential elements, while the second connection elements connect every third peak and valley of adjacent pairs of circumferential element.

In some embodiments, the curvilinear second connection elements may be flexible, or spring-like such that they are capable of contracting and expanding. The material from which the curvilinear second connection elements are made and/or the geometrical configuration of the curvilinear second connection elements and/or stent may facilitate the flexibility of the curvilinear second connection elements.

The spring-like qualities of the curvilinear second connection elements may contribute to the overall flexibility of the stent. For example, the spring-like qualities of the curvilinear second connection elements may facilitate the radial expansion and/or contraction of the stent. On some occasions, this radial expansion and/or contraction of the stent may occur such that the stent retains the same, or a substantially similar (approximately +/- 10%), lengthwise dimension as measured relative to longitudinal axis 81. In this way, a stent's radial contraction or expansion may be substantially larger when considered in proportion to the stent's longitudinal contraction or expansion.

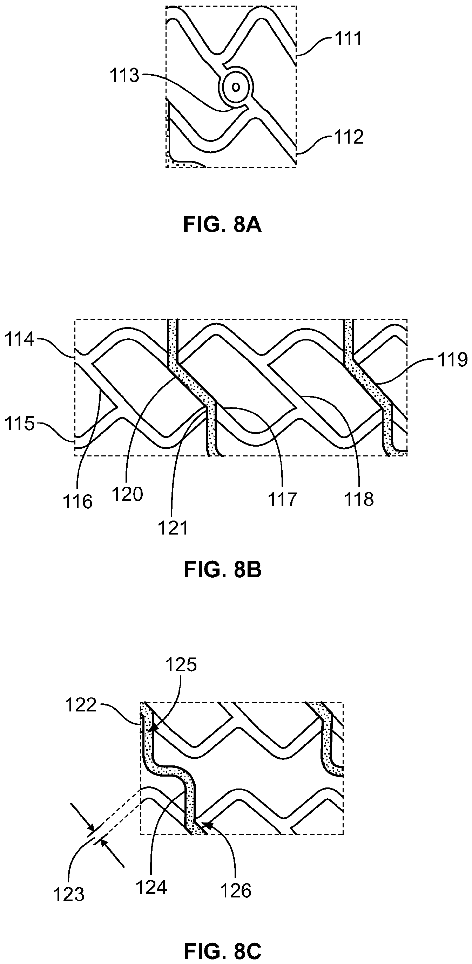

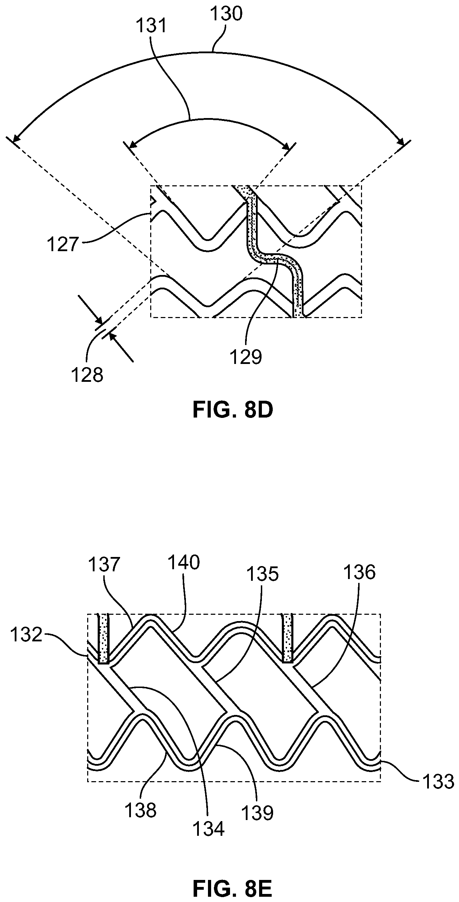

Figure 8A-8E shows a close-up view of various portions of the stent pattern shown in Figure 7. A view of one end of stent which contains a marker dot 113 is shown in Figure 8A. The circumferential elements in the pair are labeled 111, 112. Another pair of circumferential elements is shown in Figure 8B. The circumferential elements 114, 115 of the pair are connected by first connection elements 116-119 from the valley 120 of one connection element 114 of the pair to the peak 121 of the other circumferential element 115. Note, although the circumferential elements in this embodiment are in-phase with each other, the first connection elements connect adjacent valleys and peaks. Figure 8C shows the detail of the connection between adjacent pairs of circumferential elements. The circumferential elements 122, 123 in adjacent pairs of circumferential elements are connected by a curvilinear second connection element 124, which connects the peak 125 and valley 126 of the two circumferential elements. Figure 8D shows another close-up view of the circumferential elements 127, 128 between adjacent pairs of circumferential elements connected by a curvilinear second connection element 129. The angles formed by the undulations of the circumferential elements 127, 128 are shown as 130, 131. Figure 8E shows a close-up view of a pair of circumferential elements 132, 133. The circumferential elements are connected by first connection elements 134 - 136. The polygonal nature of the cells formed by the circumferential elements and connection elements is shown as 136, 137, 134, 138, 139, 135, 140.

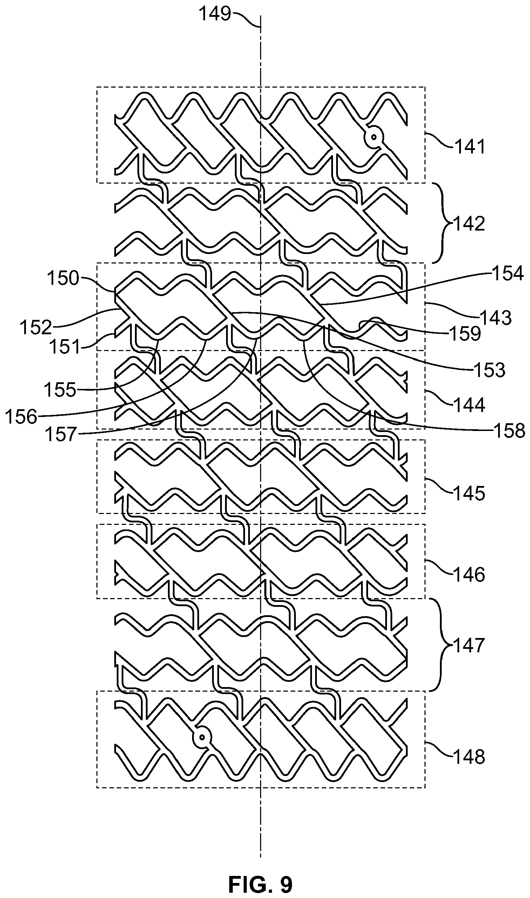

As noted in Figure 9, the number first connection elements between circumferential elements in a pair of circumferential elements can vary. In the embodiment shown, the pairs of circumferential elements are labeled 141-148 (longitudinal axis of the stent 149). Focusing on one pair, 143, the circumferential elements are labeled 150, 151. The first connection elements 152-154 are shown connecting the two circumferential elements. In contrast to the previous embodiments, the number of first connection elements is three, whereas in other embodiments shown the number of first connection elements is six. The first connection elements 152-154 are spaced evenly at every other undulation forming the circumferential element. In other words, the undulations between points of contact with the first connection elements are 155, 156, 157, 158, and 159. The radial spacing of the first connection elements around the circumference of the stent can be even, e.g., at 0°, 120°, 240°. In this embodiment, the pairs of circumferential elements at either end of the stent 141, 148, have six first connection elements.

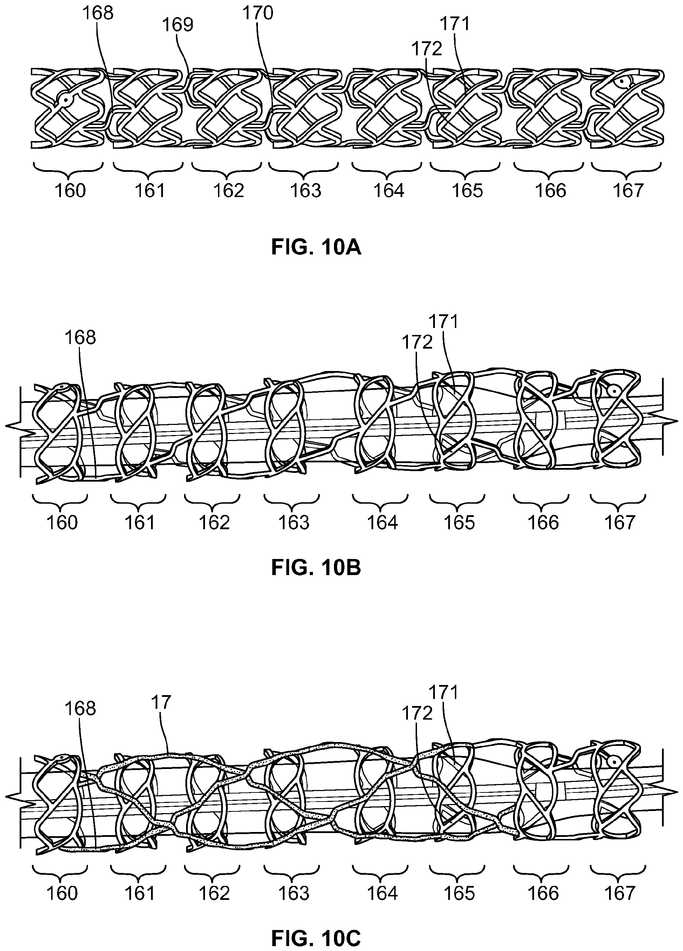

When the stent expands radially, the distance between adjacent pairs of circumferential elements increases, while the distance between circumferential elements within a pair decreases. A stent is illustrated in Figures 10A-10C, showing a three dimension figure of a stent as cut (A), and expanded (B and C). The pairs of circumferential elements are illustrated as 160-167. Selected second connection elements, 169-170 and selected first connection elements 172 are noted for illustration purposes only. When the stent is radially expanded, the curvilinear second connection element straightens, see 168 in 10A, increasing the distance between adjacent pairs of circumferential elements 160, 161 thereby forming a triple helix 168, 171, and 173. Although Figure 10B illustrates a triple helix formed by the first and connection elements, depending on the number of first and second connection elements present, stents having two, for, or more helixes may be formed.

The connection elements may contain at least one radiopaque marker. See, www.nitinol-europe.com/pdfs/stentdesign.pdf for a review of the design and makeup of radiopaque markers, which are well known in the art. The radiopaque markers may assume a variety of different sizes and shapes. For example, a radiopaque marker may contain a centrally placed marker hole. The marker hole may be circular or semicircular, but may also assume other shapes, such as a semicircular hole with an extrusion or dimple positioned at one portion of the circumference, or a hole in the shape of a heart.

The radiopaque marker may be electron-dense or x-ray refractile markers, such as metal particles or salts. Non-limiting examples of suitable marker metals include iron, gold, colloidal silver, zinc and magnesium, either in pure form or as organic compounds. Other radiopaque materials are tantalum, tungsten, platinum/iridium, or platinum. Heavy metal and heavy earth elements are useful in variety of compounds such as ferrous salts, organic iodine substances, bismuth or barium salts, etc. Further embodiments that may be utilized may encompass natural encapsulated iron particles such as ferritin that may be further cross-linked by cross-linking agents. Ferritin gel can be constituted by cross-linking with low concentrations (0.1-2%) of glutaraldehyde. The radiopaque marker may be constituted with a binding agent of one or more biodegradable polymer, such as PLLA, PDLA, PLGA, PEG, etc. In one embodiment comprising a radiopaque marker, iron containing compounds or iron particles may be encapsulated in a PLA polymer matrix to produce a pasty substance, which can be injected or otherwise deposited in the hollow receptacle contained about the stent.

The stents may also have a transition zone between the end zone and the main body. The transition zone may be formed from a plurality of undulations, each undulation comprising two adjacent struts connected by a loop with the width of the loop varying across the transition zone. The transition zone may comprise a plurality of polygons where the surface area of adjacent polygons in the transition zone increases circumferentially. U.S. Patent Publication No. 20110125251. The transition zone may take other suitable configurations.

The dimensions of the stent may vary from about 10 mm to about 300 mm in length, from 20 mm to about 300 mm in length, from about 40 mm to about 300 mm in length, from about 20 mm to about 200 mm in length, from about 60 mm to about 150 mm in length, or from about 80 mm to about 120 mm in length. In one embodiment, the stent may be about 88.9 mm in length. The internal diameter (I.D.) of the stent may range from about 2 mm to about 25 mm, from about 2 mm to about 5 mm (e.g., for the coronary arteries), from about 4 mm to about 8 mm (e.g., for neurological spaces in the CNS, both vascular and nonvascular), from about 6 mm to about 12 mm (e.g., for the iliofemoral), from about 10mm to about 20 mm (e.g., for the ilioaortic) and from about 10 mm to about 25 mm (e.g., for the aortic).

The device of the present invention may be used as a self-expanding stent or with any balloon catheter stent delivery system, including balloon catheter stent delivery systems described in U.S. Patent Nos. 6,168,617, 6,222,097, 6,331,186 and 6,478,814. In one embodiment, the present device is used with the balloon catheter system disclosed in U.S. Patent No. 7,169,162.

The device of the present invention may be used with any suitable catheter, the diameter of which may range from about 0.8mm to about 5.5 mm, from about 1.0 mm to about 4.5 mm, from about 1.2 mm to about 2.2 mm, or from about 1.8 to about 3 mm. In one embodiment, the catheter is about 6 French (2 mm) in diameter. In another embodiment, the catheter is about 5 French (1.7 mm) in diameter.

The stent may be inserted into the lumen of any vessel or body cavity expanding its cross-sectional lumen. The invention may be deployed in any artery, vein, duct or other vessel such as a ureter or urethra and may be used to treat narrowing or stenosis of any artery, including, the coronary, infrainguinal, aortoiliac, subclavian, mesenteric or renal arteries. Other types of vessel obstructions, such as those resulting from a dissecting aneurysm are also encompassed by the invention.

The subjects that can be treated using the stent and methods of this invention are mammals, including a human, horse, dog, cat, pig, rabbit, rodent, monkey and the like.

The stent of the present invention may be formed from at least one bioabsorbable polymer representing a wide range of different polymers. Typically, bioabsorbable polymers comprise aliphatic polyesters based on lactide backbone such as poly L-lactide, poly D-lactide, poly D, L-lactide, mesolactide, glycolides, lactones, as homopolymers or copolymers, as well as formed in copolymer moieties with co-monomers such as, trimethylene carbonate (TMC) or ε-caprolactone (ECL). U.S. Patent Nos. 6,706,854 and 6,607,548; EP 0401844; and Jeon et al. Synthesis and Characterization of Poly (L-lactide) - Poly (ε-caprolactone). Multiblock Copolymers Macromolecules 2003: 36, 5585 - 5592. The copolymers comprises a moiety such as L-lactide or D-lactide of sufficient length that the copolymer can crystallize and not be sterically hindered by the presence of glycolide, polyethylene glycol (PEG), ε-caprolactone, trimethylene carbonate or monomethoxy-terminated PEG (PEG-MME). For example, in certain embodiments greater than 10, 100 or 250 L or D-lactides may be arrayed sequentially in a polymer. The stent may also be composed of bioabsorbable polymeric compositions such as those disclosed in U.S. Patent No. 7,846,361 and applicant's co-pending U.S. Patent Publication No. 2010/0093946.

The following nomenclature will now be used with the polymer nomenclature being based on the presence of the monomer type.

| | |
|---|---|
| LPLA: | Poly(L-lactide) |
| LPLA-PEG: | Poly(poly-L-lactide-polyethylene glycol) |
| DPLA: | Poly(D-lactide) |
| DPLA-TMC: | Poly(poly D-lactide-co-trimethylene carbonate) |
| DLPLA: | Poly(DL-lactide), a racemic copolymer D-co-L-lactide |
| LDPLA: | Poly(L-co-D-lactide) |
| LDLPLA: | Poly(L-co-DL-lactide), named for the method of monomer introduction |
| PGA: | Poly(glycolide) |
| PDO: | Poly(dioxanone) (PDS is Trademark) |
| SR: | "Self reinforced" (a processing technique) |
| TMC: | Trimethylene carbonate |
| PCL: | Poly(ε-caprolactone) |
| LPLA-TMC: | Poly(poly L-lactide-co-trimethylene carbonate) |
| LPLG: | Poly(L-lactide-co-glycolide) |
| POE: | Poly Orthoester |

In an embodiment of the present invention, the composition comprises a base polymer of poly(L-lactide) or poly(D-lactide). Advantageous base polymer compositions include blends of poly(L-lactide) and poly(D-lactide). Other advantageous base polymer compositions include poly(L-lactide-co-D,L-lactide) or poly(D-lactide-co-D,L-lactide) with a D,L-lactide co-monomer molar ratio from 10 to 30%, and poly(L-lactide-co-glycolide) or poly(D-lactide-co-glycolide) with a glycolide co-monomer molar ratio from 10 to 20%.

Another embodiment embodies a base polymer featuring a poly (L-lactide) moiety, and/or a poly (D-lactide) moiety, linked with a modifying copolymer thereof, including poly (L-lactide-co-tri-methylene-carbonate or poly(D-lactide-co-tri-methylene-carbonate) and (L-lactide-co-ε-caprolactone), or poly(D-lactide-co-ε-caprolactone), in the form of block copolymers or blocky random copolymers, wherein the lactide chain length is sufficient to affect cross-moiety crystallization.

In another embodiment, the polymer composition allows the development of the lactide racemate (stereo complex) crystal structure, between the L and D moieties, to further enhance the mechanical properties of the bioabsorbable polymer medical device. The formation of the racemate (stereo complex) crystal structure can accrue from formulations such as combinations of:
Poly L-lactide with Poly D-lactide with Poly L-lactide-co-TMC;
Poly D-lactide with Poly L-lactide-co-TMC;
Poly L-lactide with Poly D-lactide-co-TMC;
Poly L-lactide with Poly D-lactide with Poly D-lactide-co-TMC;
Poly L-lactide-co-PEG with Poly D-lactide-co-TMC; and
Poly D-lactide-co-PEG with Poly L-lactide-co-TMC.

Poly-lactide racemate compositions of this embodiment may have an especially advantageous characteristic in being "cold formable or bendable" without adding heat. Cold-bendable scaffolds of the invention do not require beating to become flexible enough to be crimped onto a carrier device or accommodate irregularly shaped organ spaces. Cold bendable ambient temperatures are defined as room temperature not exceeding 30°C. Cold-bendable scaffolds, for example, afford sufficient flexibility when implanted allowing for an expanded scaffold device in an organ space such as pulsating vascular lumen. For example, in terms of a stent, it may be desirable to utilize polymeric compositions that afford mostly amorphous polymer moieties after fabrication that can crystallize particularly when the secondary nested or end-positioned meandering struts when the scaffold is strained by stretching upon balloon expansion for implantation. Such cold-bendable polymeric scaffold embodiments are not brittle and do not have to be preheated to a flexible state prior to implantation onto a contoured surface space in the body. Cold-bendability allows these blends to be crimped at room temperature without crazing, and moreover, the blends can be expanded at physiological conditions without crazing.

Poly-lactide racemate compositions and non-racemate compositions of embodiments herein may be processed to have blocky moieties allowing cross moiety crystallization even with the addition of an impact modifier to the blend composition. Such a blend introduces the possibility to design device specific polymer compositions or blends by producing either single or double Tg's (glass melt transition points).

Poly-lactide racemate compositions may show significant improvement in re-crystallization capability over, for example, non-racemate PLDL-lactide blends. An advantageous racemate alignment of the different polylactide moieties can be achieved, for example, by blending a poly-D-lactide with the copolymer poly L-lactide-co-TMC capable of forming a racemate crystal across the different polylactide stereomoieties, for example, without limitation, when stretched during expansion to the required emplacement diameter. This strain induced crystallization, without adverse crazing, results in an increase of the mechanical properties reflected also in a positive change of modulus data over the base of the base materials.

Cross moiety crystallization of compositions with copolymers appears to be limited to copolymer with monomer molar ratios ranging from about 90:10 through 50:50. In fact, at a molar ratio of 50:50, the polymer moieties sterically impeded crystallization whereas the greater ratios are much more suitable for cross moiety crystallization. On the basis of experimental induced crystallization, different blends with various concentrations of lactide copolymers such as TMC, to which an excess of poly (D-lactide) for racemate alignment with the L-lactide component has been added, the effective concentration of the copolymer in a racemate composition may be equal to, or less than, 40%. Thus, the thermal cross-links formed by cross moiety crystallization serves to reduce elongation or creep while maintaining the intended toughening mechanism. The advantageously strong racemate composition affords increased modulus data in tensile tests avoiding the method for reducing the tensile strength in the polymer blend.

An advantageous racemate composition embodiment provides a bioabsorbable polymer with minimal degradation in terms of high residual monomer level such that the contaminant monomeric residual fraction does not exceed about 0.5%, or preferably not in excess of about 0.3%. In an embodiment a concentration of monomeric contaminant of the polymer of the present invention is as low as about 0.2%.

Polymer compositions of embodiments described herein may comprise a base polymer present from about 70% to 95% by weight, or from about 70% to 80% by weight of the composition. For example, in one embodiment, the polymer formulation may comprise from about 70% by weight poly L-lactide (about 2.5 to 3 IV) with the poly L-lactide-co-TMC (70/30 mole/mole) (1.4 to 1.6 IV). In another embodiment, the polymer formulation may comprise 70% by weight triblock poly L-lactide-co-PEG (99/01 mole/mole) (2.5 to 3 IV) with the poly L-lactide-co-TMC (70/30 mole/mole) (1.4 to 1.6 IV). Furthermore, the polymer composition may comprise a formulation of about 70% by weight diblock poly L-lactide-co-PEG-MME (95/05 mole/mole) (2.5 to 3 IV) with poly L-lactide-co-TMC (70/30 mole/mole) (1.4 to 1.6 IV). Other embodiments provide formulations wherein ε-caprolactone is substituted in a composition for the aforementioned TMC. Similarly, an embodiment may provide formulations wherein PEG-MME may be substituted for PEG.

As is understood in this art, polymer compositions of the present invention can be customized to accommodate various requirements of the selected medical device. The requirements include mechanical strength, elasticity, flexibility, resilience, and rate of degradation under physiological and localized anatomical conditions. Additional effects of a specific composition concern solubility of metabolites, hydrophilicity and uptake of water and any release rates of matrix attached or enclosed pharmaceuticals.

The polymer implant utility can be evaluated by measuring mass loss, decrease in molecular weight, retention of mechanical properties, and/or tissue reaction. More critical for scaffold performance are hydrolytic stability, thermal transitions crystallinity and orientation. Other determinants negatively affecting scaffold performance include, but not exclusively, monomeric impurities, cyclic and acyclic oligomers, structural defects and aging.

The medical device fashioned from the polymer compositions above may be significantly amorphous post extrusion or molding. Such devices may be subjected to controlled re-crystallization to induce incremental amounts of crystallinity and mechanical strength enhancement. Further crystallization can be induced by strain introduction at the time of device deployment. Such incremental re-crystallization may be employed either on a device "blank" prior to secondary or final fabrication (such as by laser cutting) or post such secondary fabrication. Crystallization (and thus mechanical properties) can also be maximized by strain induction such as by "cold" drawing polymeric tubing, hollow fiber, sheet or film, or monofilament prior to further fabrication. Crystallinity has been observed to contribute a greater stiffness in the medical device. Therefore, the polymer composition and steric complex of the scaffold has both amorphous and paracrystalline moieties. The initially semicrystalline polymer portion can be manipulated by the action of stretching or expansion of a given device. Yet an adequate amount of amorphous polymeric character is desirable for flexibility and elasticity of the polymeric device. The usual monomer components include lactide, glycolide, caprolactone, dioxanone, and trimethylene carbonate. The stent may also be constructed to allow relatively uniform exposure to local tissue or circulatory bioactive factors and enzymes perfusing and acting on the polymer structure during bioabsorption.

Advantageously, the rate of in situ breakdown kinetics of the polymeric matrix of an organ space implant, such as a cardiovascular stent, is sufficiently gradual to avoid tissue overload, inflammatory reactions or other more adverse consequences. In an embodiment, the scaffold is fabricated to survive at least one month.

The pharmaceutical compositions may be incorporated within the polymers by, for example, grafting to the polymer active sites or coating. An embodiment of the polymer according to the invention affords attachment or incorporation the biological healing factors or other drugs in the polymeric matrix or a polymer coating.

In another embodiment, the composition may be constructed to structurally enclose or attach to drugs in the polymeric matrix. The purpose of such additives may to provide, for example with respect to a stent, treatment of the cardiovascular system or in vascular site in contact with the medical device polymer. The kind of enclosure or attachment of drugs in the polymer may determine the rate of release from the device. For example, the drug or other additive may be bound in the polymer matrix by various known methods including, but not limited to, covalent bonds, non-polar bonds as well as an ester or similar bioreversible bonding means.

In one embodiment, a bioabsorbable implantable medical device may be covered with a biodegradable and bioabsorbable coating containing one or more barrier layers where the polymer matrix contains one or more of the aforementioned pharmaceutical substances. In this embodiment, the barrier layer may comprise a suitable biodegradable material, including but not limited to, suitable biodegradable polymers including: polyesters such as PLA, PGA, PLGA, PPF, PCL, PCC, TMC and any copolymer of these; polycarboxylic acid, polyanhydrides including maleic anhydride polymers; polyorthoesters; poly-amino acids; polyethylene oxide; polyphosphacenes; polylactic acid, polyglycolic acid and copolymers and mixtures thereof such as poly(L-lactic acid) (PLLA), poly(D,L-lactide), poly(lactic acid-co-glycolic acid), 50/50 (DL-lactide-co-glycolide); polydixanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethyl-carbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate. The number of barrier layers that the polymeric scaffold on a device may have depends on the amount of therapeutic need as dictated by the therapy required by the patient. For example, the longer the treatment, the more therapeutic substance required over a period of time, the more barrier layers to provide the pharmaceutical substance in a timely manner.

In another embodiment, the additive in the polymer composition may be in the form of a multiple component pharmaceutical composition within the matrix such as containing a last release pharmaceutical agent to retard early neointimal hyperplasia/smooth muscle cell migration and proliferation, and a secondary biostable matrix that releases a long acting agent for maintaining vessel patency or a positive blood vessel remodeling agent, such as endothelial nitric oxide synthase (eNOS), nitric oxide donors and derivatives such as aspirin or derivatives thereof, nitric oxide producing hydrogels, PPAR agonist such as PPAR-α gands, tissue plasminogen activator, statins such as atorvastatin, erythropoietin, darbepotin, serine proteinase-1 (SERP-1) and pravastatin, steroids, and/or antibiotics.

Pharmaceutical compositions may be incorporated into the polymers or may be coated on the surface of the polymers after mixing and extrusion by spraying, dipping or painting or microencapsulated and then blended into the polymer mixture. U.S. Patent No. 6,020,385. If the pharmaceutical compositions are covalently bound to the polymer blend, they may be linked by hetero- or homo-bifunctional cross linking agents (see, http://www.piercenet.com/products/browse.cfm?fldID=020306). Pharmaceutical agents that may be incorporated into the stents or may be coated on the stent include, but are not limited to, (i) pharmacological agents such as, (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, thymidine kinase inhibitors, rapamycin, 40-0-(2- Hydroxyethyl)rapamycin (everolimus), 40-0-Benzyl-rapamycin, 40-0(4'-Hydroxymethyl)benzyl-rapamycin, 40-0-[4'-(1,2-Dihydroxyethyl)]benzyl-rapamycin, 40-Allyl-rapamycin, 40-0-[3'-(2,2-Dimethyl-1,3-dioxolan-4(S)-yl-prop-2'-en-1'-yl]-20 rapamycin, (2':E,4'S)-40-0-(4',5'.:Dihydroxypent-2'-en-1'-yl),r apamycin 40-0(2Hydroxy) ethoxycarbonylmethyl-rapamycin, 40-0-(3- Hydroxypropyl-rapamycin 40-0-((Hydroxy)hexyl-rapamycin 40-0-[2-(2-Hydroxy)ethoxy]ethyl-rapamycin, 40-0-[(3S)-2,2Dimethyldioxolan-3-yl]methyl-rapamycin, 40-0-[(2S)-2,3- Dihydroxyprop-1-yl]-rapamycin, 40-0-(2- Acctoxy)ethyl-rapamycin, 40-0-(2-Nicotinoyloxy)ethyl-rapamycin, 40-0-[2-(N-25 Morpholino) acetoxyethyl-rapamycin, 40-0-(2-N-Imidazolylacetoxy)ethyl-rapamycin, 40-0[2-(N-Methyl-N'-piperazinyl)acetoxy]ethyl-rapamycin, 39-0-Desmethyl-3.9,40-0,0 ethylene-rapamycin, (26R)-26- Dihydro-40-0-(2-hydroxy)ethyl-rapamycin, 28-O Methyrapamycin, 40-0-(2-Aminoethyl)-rapamycin, 40-0-(2-Acetaminoethyl)-rapamycin 40-0(2-Nicotinamidoethyl)-rapamycin, 40-0-(2-(N-Methyl- imidazo- 2' ylcarbcthoxamido)ethyl)- 30 rapamycin, 40-0-(2-Ethoxycarbonylaminoethyl)-rapamycin, 40-0-(2-Tolylsulfonamidoethyl)- rapamycin, 40-0-[2-(4',5'-Dicarboethoxy-1',2';3'-triazol-1'-yl)-ethyl]rapamycin, 42-Epi-(telrazolyl)rapamycin (tacrolimus), and 42-[3-hydroxy-2-(hydroxymethyl)-2-methylpropanoate]rapamycin (temsirolimus) (WO2008/086369); (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e. g. , tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; and, (o) agents that interfere with endogenous vasoactive mechanisms, (ii) genetic therapeutic agents include anti-sense DNA and RNA as well as DNA coding for (a) anti-sense RNA, (b) tRNA or rRNA to replace defective or deficient endogenous molecules, (b) angiogenic factors including growth factors such as acidic and basic fibroblast growth factors, vascular endothelial growth factor, epidermal growth factor, transforming growth factor a and P, platelet-derived endothelial growth factor, platelet-derived growth factor, tumor necrosis factor a, hepatocyte growth factor and insulin-like growth factor, (c) cell cycle inhibitors including CD inhibitors, and (d) thymidine kinase ("TK") and other agents useful for interfering with cell proliferation.

Other pharmaceutical agents that may be incorporated into the stents include, but are not limited to, acarbosc, antigens, beta-receptor blockers, non-steroidal antiinflammatory drugs (NSAID, cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomcthasone, betahistine, bezafibrate, bicalutamide, diazepam and diazepam derivatives, budesonide, bufexamac, buprcnorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidinc, cotrimoxazole, codeine, caffeine, vitamin D and derivatives of vitamin D, colestyramine, cromoglicic acid, coumarin and coumarin derivatives, cysteine, cytarabine, cyclophosphamide, cyclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, erythropoietin and erythropoietin derivatives, morphinans, calcium antagonists, irinotecan, modafmil, orlistat, peptide antibiotics, phenytoin, riluzoles, risedronate, sildenafil, topiramatc, macrolide antibiotics, oestrogen and oestrogen derivatives, progestogen and progestogen derivatives, testosterone and testosterone derivatives, androgen and androgen derivatives, ethenzamide, etofenamate, ctofibrate, fcnofibrate, etofylHne, etoposide, famciclovir, famotidine, felodipine, fenoftbrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mcpindolol, meprobamate, meropenem, mesalazinc, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastinc, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tctracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazolinc, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobutcrol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorclbinc, vinpocetine, viquidil, warfarin, xantinol nicotinate, xipamide, zafirlukast, zalcitabine, zidovudine, zolmitriptan, Zolpidem, zoplicone, zotipine and the like. See, e.g., U.S. Patent Nos. 6,897,205, 6,838,528 and 6,497,729.

The stent may also be coated with at least one type of antibodies. For example, the stent may be coated with antibodies or polymeric matrices which are capable of capturing circulating endothelial cells. U.S. Patent No. 7,037,772 (see also, U.S. Patent Publications Nos. 20070213801,200701196422,20070191932,20070156232,20070141107,20070055367, 20070042017,20060135476,20060121012).

The stent of the present invention may also be formed from metal such as nickel-titanium (Ni-Ti). A metal composition and process of manufacturing the device is disclosed in U.S. Patent No. 6,013,854. The super elastic metal for the device is preferably a super elastic alloy. A super elastic alloy is generally called "a shape-memory alloy" and resumes its original shape after being deformed to such a degree that an ordinary metal undergoes permanent deformation. Super elastic alloys useful in the invention include: Elgiloy® and Phynox® spring alloys (Elgiloy® alloy is available from Carpenter Technology Corporation of Reading Pa.; Phynox® alloy is available from Metal Imphy of Imphy, France), 316 stainless steel and MP35N alloy which are available from Carpenter Technology corporation and Latrobe Steel Company of Latrobe, Pa., and superelastic Nitinol nickel-titanium alloy which is available from Shape Memory Applications of Santa Clara, Calif. U.S. Patent No. 5,891,191.

The device of the present invention may be manufactured in numerous ways. The device may be formed from a tube by removing various portions of the tube's wall to form the patterns described herein. The resulting device will thus be formed from a single contiguous piece of material, eliminating the need for connecting various segments together. Material from the tube wall may be removed using various techniques including laser (YAG laser for example), electrical discharge, chemical etching, metal cutting, a combination of these techniques, or other well known techniques. U.S. Patent Nos. 5,879,381 and 6,117,165 which are hereby incorporated in their entirety by reference. Forming stents in this manner allows for creation of a substantially stress-free structure. In particular, the length may be adapted to that of the diseased part of the lumen in which the stent is to be placed. This may avoid using separate stents to cover the total diseased area.

In an alternate embodiment, a method for fabricating a tube-shaped stent may include preparing a racemic poly-lactide mixture and fabricating a biodegradable polymer tube of the racemic poly-lactide mixture and laser cutting the tube until such scaffold is formed. In this embodiment, the fabrication of the scaffold can be performed using a molding technique, which is substantially solvent-free, or an extrusion technique.

Reference is also made, to U.S. Patent Nos. 7,329,277, 7,169,175, 7,846,197, 7,846,361, 7,833,260, 6,0254,688, 6,254,631, 6,132,461, 6,821,292, 6,245,103 and 7,279,005. In addition, U.S. Patent Application Nos. 11/781,230, 12/507,663, 12/508,442, 12/986,862, 11/781,233, 12/434,596, 11/875,887, 12/464,042, 12/576,965, 12/578,432, 11/875,892, 11/781,229, 11/781,353, 11/781,232, 11/781,234, 12/603,279, 12/779,767 and 11/454,968, as well as U.S. Patent Publication No. 2001/0029397, are also referred to. The scope of the present invention is not limited by what has been specifically shown and described hereinabove. Those skilled in the art will recognize that there are suitable alternatives to the depicted examples of materials, configurations, constructions and dimensions. Numerous references, including patents and various publications, are cited and discussed in the description of this invention. The citation and discussion of such references is provided merely to clarify the description of the present invention and is not an admission that any reference is prior art to the invention described herein. Variations, modifications and other implementations of what is described herein will occur to those of ordinary skill in the art without departing from the spirit and scope of the invention. While certain embodiments of the present invention have been shown and described, it will be obvious to those skilled in the art that changes and modifications may be made without departing from the scope of the invention. The matter set forth in the foregoing description and accompanying drawings is offered by way of illustration only and not as a limitation.

## Claims

1. A stent comprising a generally cylindrically shaped main body formed from at least one bioabsorbable polymer and having a plurality of expandable circumferential elements, wherein said circumferential elements each comprise a plurality of undulations having alternating peaks and valleys, wherein adjacent circumferential elements (7..18) form a pair (1..6) connected by at least one first connection element (19..24) and adjacent pairs of circumferential segments are connected by at least one second connection element (25..27), and wherein the first connection elements (39,42) and second connection elements (40,43,45) together with portions of the circumferential elements (41,44,46) form a substantially helical pattern (36..38) across the longitudinal axis (35) of the stent, **characterized in that** said first connection elements (19..24) connect a peak (31) in a first one of said pair of circumferential elements to a valley (30) in a second one of said pair of circumferential elements, and **in that** said second connections elements (25..27) connect a peak-to-peak (32,33), valley-to-peak of valley-to-valley, valley-to-peak or peak-to-peak between in first pair of circumferential elements and an adjacent pair of circumferential elements.

2. The stent of claim 1, **characterized in that** said first connection elements connect each peak in said first one of said pair of circumferential elements to a valley in said second one of said pair of circumferential elements, while said second connections elements connect every third peak and valley of adjacent pairs of circumferential elements.

3. The stent of claim 1, **characterized in that** said first connection elements connect every third peak in said first one of said pair of circumferential elements to a valley in said second one of said pair of circumferential elements.

4. The stent of claim 1, wherein the second connection elements are curvilinear.

5. The stent of claim 3, wherein there are six first connection elements within each pair of circumferential elements.

6. The stent of claim 2, wherein there are three first connection elements within each pair of circumferential elements.

7. The stent of claim 1, wherein the second connection elements are linear.

8. The stent of claim 1, wherein the circumferential elements in each pair are in phase with each other.

9. The stent of claim 1, wherein the second connection element connects two adjacent pairs of circumferential elements from a valley of one undulation in one circumferential element in a first pair to a peak of one undulation in a second circumferential element in a second adjacent pair.

10. The stent of claim 1, wherein the circumferential elements are formed from undulations having a sinusoidal pattern.

11. The stent of claim 1, wherein a first connection element further comprises at least one radiopaque marker.

12. The stent of claim 1, wherein the stent is crimped.

13. The stent of claim 1, wherein the stent is radially expanded.

14. The stent of claim 1, wherein the second connection elements are curvilinear and become substantially linear after expansion of the stent.

15. The stent of claim 14, wherein a distance between adjacent pairs of circumferential elements increases and the overall length of the stent remains substantially constant.

16. The stent of claim 1, wherein a distance between adjacent pairs of circumferential elements decreases and the overall length of the stent remains substantially constant when compressed.

17. The stent of claim 1, wherein said pairs of adjacent circumferential elements comprise a plurality of polygons organized as a plurality of circumferential components, wherein adjacent circumferential components are connected by at least one second connection element and form a substantially helical pattern across the longitudinal axis of the stent.

18. The stent of claim 17, wherein adjacent circumferential components are connected by three second connection elements.

## Patentansprüche

1. Stent, umfassend einen im Wesentlichen zylindrischen Hauptkörper, gebildet aus wenigstens einem bioabsorbierbaren Polymer, mit einer Mehrzahl von expandierbaren Umfangselementen; jedes der Umfangselemente umfasst eine Mehrzahl von Wellen mit abwechselnden Gipfeln und Tälern;
einander benachbarte Umfangselemente (7 ... 18) bilden ein Paar (1 ... 6), verbunden durch wenigstens ein erstes Verbindungselement (19 ... 24) und einander benachbarte Paare von Umfangssegmenten sind durch wenigstens ein zweites Verbindungselement (25 ... 27) miteinander verbunden; die ersten Verbindungselemente (39, 42) und die zweiten Verbindungselemente (40, 43, 45) zusammen mit Teilen der Umfangselemente (41, 44, 46) bilden im Wesentlichen spiralige Muster (36 ... 38) über die Längsachse (35) des Stent, **dadurch gekennzeichnet, dass** die ersten Verbindungselemente (19 ... 24) einen Gipfel (31) in einem ersten Paar der Umfangselemente mit einem Tal (30) eines zweiten Paares von Umfangselementen verbinden, und dass die zweiten Verbindungselemente (25 ... 27) einen ersten Gipfel-zu-Gipfel (32, 33), Tal-zu-Gipfel des Tal-zu-Tal, Tal-zu-Gipfel oder Gipfel-zu-Gipfel zwischen einem ersten Paar von Umfangselementen und einem benachbarten Paar von Umfangselementen miteinander verbinden.

2. Stent nach Anspruch 1, **dadurch gekennzeichnet, dass** die ersten Verbindungselemente jeden Gipfel in dem ersten Paar von Umfangselementen mit einem Tal im zweiten des Paares von Umfangselementen verbinden, während die zweiten Verbindungselemente jeden dritten Gipfel und Paar einander benachbarter Paare von Umfangselementen miteinander verbinden.

3. Stent nach Anspruch 1, **dadurch gekennzeichnet, dass** die ersten Verbindungselemente jeden dritten Gipfel in einem ersten des Paares von Umfangselementen mit einem Tal in einem zweiten des Paares von Umfangselementen verbinden.

4. Stent nach Anspruch 1, wobei die zweiten Verbindungselemente kurvenlinear sind.

5. Stent nach Anspruch 3, wobei sechs erste Verbindungselemente innerhalb jedes Paares von Umfangselementen liegen.

6. Stent nach Anspruch 2, wobei drei erste Verbindungselemente innerhalb eines jeden Paares von Umfangselementen liegen.

7. Stent nach Anspruch 1, wobei die zweiten Verbindungselemente linear verlaufen.

8. Stent nach Anspruch 1, wobei die Umfangselemente in jedem Paar in Phase miteinander liegen.

9. Stent nach Anspruch 1, wobei die zweiten Verbindungselemente zwei einander benachbarte Paare von Umfangselementen von einem Gipfel einer Welle in einem Umfangselement in einem ersten Paar mit einem Gipfel einer Welle in einem zweiten Umfangselement in einem zweiten Paar verbinden.

10. Stent nach Anspruch 1, wobei die Umfangselemente aus Wellen mit sinusähnlichem Muster gebildet sind.

11. Stent nach Anspruch 1, wobei ein erstes Verbindungselement weiterhin wenigstens einen radiopaken Marker umfasst.

12. Stent nach Anspruch 1, wobei der Stent gekrümmt ist.

13. Stent nach Anspruch 1, wobei der Stent radial expandiert ist.

14. Stent nach Anspruch 1, wobei die zweiten Verbindungselemente kurvenlinear sind und nach dem Expandieren des Stents im Wesentlichen gradlinig werden.

15. Stent nach Anspruch 14, wobei ein Abstand zwischen einander benachbarten Paaren von Umfangselementen zunimmt und die Gesamtlänge des Stents im Wesentlichen gleich bleibt.

16. Stent nach Anspruch 1, wobei ein Abstand zwischen einander benachbarten Paaren von Umfangselementen abnimmt und die Gesamtlänge des Stents beim Komprimieren im Wesentlichen gleich bleibt.

17. Stent nach Anspruch 1, wobei die Paare von einander benachbarten Umfangselementen eine Mehrzahl von Polygonen umfasst, als Mehrzahl von Umfangskomponenten ausgebildet, wobei einander benachbarte Umfangskomponenten durch wenigstens ein zweites Verbindungselement miteinander verbunden sind und ein im Wesentlichen spiraliges Muster über die Längsachse des Stents bilden.

18. Stent nach Anspruch 17, wobei einander benachbarte Umfangskomponenten durch drei zweite Verbindungselemente miteinander verbunden sind.

## Revendications

1. Un stent comprenant un corps principal de forme généralement cylindrique composé d'au moins un polymère bioabsorbable et comprenant une pluralité d'éléments périphériques expansibles, dans lequel lesdits éléments périphériques comprennent chacun une pluralité d'ondulations présentant une alternance de crêtes et de creux, les éléments périphériques adjacents (718) formant une paire (1..6) reliée par au moins un premier élément de liaison (19..24) et les paires adjacentes de segments périphériques étant reliées par au moins un second élément de liaison (25..27), et les premiers éléments de liaison (39, 42) et les seconds éléments de liaison (40, 43, 45) associés à des portions des éléments périphériques (41, 44, 46) formant un motif substantiellement hélicoïdal (36..38) le long de l'axe longitudinal (35) du stent, **caractérisé en ce que** lesdits premiers éléments de liaison (19..24) relient une crête (31) dans une première desdites paires d'éléments périphériques à un creux (30) dans une seconde desdites paires d'éléments périphériques, et **en ce que** lesdits seconds éléments de liaison (25..27) assurent une liaison crête-à-crête (32, 33), creux-à-crête ou creux-à-creux, creux-à-crête ou crête-à-crête entre une première paire d'éléments périphériques et une paire adjacente d'éléments périphériques.

2. Le stent de la revendication 1, **caractérisé en ce que** les premiers éléments de liaison qui relient chaque crête dans ladite première paire desdites paires d'éléments périphériques à un creux dans ladite seconde paire de ladite paire d'éléments périphériques, tandis que lesdits seconds éléments de liaison relient toutes les troisièmes crêtes et tous les troisièmes creux des paires adjacentes d'éléments périphériques.

3. Le stent de la revendication 1, **caractérisé en ce que** lesdits premiers éléments de liaison relient chaque troisième crête dans ladite première des paires d'éléments périphériques à un creux dans ladite seconde desdites paires d'éléments périphériques.

4. Le stent de la revendication 1, dans lequel les seconds éléments de liaison sont curvilinéaires.

5. Le stent de la revendication 3, dans lequel il y a six premiers éléments de liaison à l'intérieur de chaque paire d'éléments périphériques.

6. Le stent de la revendication 2, dans lequel il y a trois premiers éléments de liaison à l'intérieur de chaque paire d'éléments périphériques.

7. Le stent de la revendication 1, dans lequel les seconds éléments de liaison sont linéaires.

8. Le stent selon la revendication 1, dans lequel les éléments périphériques dans chaque paire sont en phase les uns par rapport aux autres.

9. Le stent de la revendication 1, dans lequel le second élément de liaison relie deux paires adjacentes d'éléments périphériques depuis un creux d'une ondulation dans un élément périphérique dans une première paire à une crête d'une ondulation dans un second élément périphérique dans une seconde paire adjacente.

10. Le stent selon la revendication 1, dans lequel les éléments périphériques sont formés d'ondulations présentant un motif sinusoïdal.

11. Le stent selon la revendication 1, dans lequel un premier élément de liaison comprend également au moins un marqueur radioopaque.

12. Le stent de la revendication 1, dans lequel le stent est serti.

13. Le stent selon la revendication 1, dans lequel le stent est déployé radialement.

14. Le stent de la revendication 1, dans lequel les seconds éléments de liaison sont curvilinéaires et deviennent substantiellement linéaires après le déploiement du stent.

15. Le stent de la revendication 14, dans lequel une distance entre des paires adjacentes d'éléments périphériques augmente et la longueur globale du stent reste substantiellement constante.

16. Le stent selon la revendication 1, dans lequel une distance entre des paires adjacentes d'éléments périphériques diminue et la longueur moyenne du stent reste substantiellement constante lorsqu'il est comprimé.

17. Le stent de la revendication 1, dans lequel lesdites paires d'éléments périphériques adjacents comprennent une pluralité de polygones organisés comme une pluralité de composants périphériques, dans lequel les composants périphériques sont reliés par au moins un second élément de liaison et forment un motif substantiellement hélicoïdal le long de l'axe longitudinal du stent.

18. Le stent selon la revendication 17, dans lequel les composants périphériques adjacents sont reliés par trois seconds éléments de liaison.
